# EUROPÄISCHE PATENTSCHRIFT

(11) **EP 0 469 481 B1**
(45) Veröffentlichungstag und Bekanntmachung des Hinweises auf die Patenterteilung: **08.03.1995**
(21) Anmeldenummer: 91112596.1
(22) Anmeldetag: 26.07.1991
(51) Int. Cl.: C07D 498/10, C07D 498/20, C08K 5/35

(54) **Verfahren zur Herstellung von 1-Oxa-3, 8-diaza-4-oxo-spiro-[4,5]decan-Verbindungen**
Process for the preparation of 1-oxa-3,8-diaza-4-oxo-spiro-[4,5]decane compounds
Procédé pour la préparation de composés de 1-oxa-3,8-diaza-4-oxo-spiro-[4,5]décane

(30) Priorität: 01.08.1990 DE 4024415
(43) Veröffentlichungstag der Anmeldung: 05.02.1992
(73) Patentinhaber: HOECHST AKTIENGESELLSCHAFT, 65926 Frankfurt am Main (DE)
(72) Erfinder: Schmailzl, Georg, Dr., W-8906 Gersthofen (DE)

(56) Entgegenhaltungen:
- EP-A- 0 208 263
- DE-A- 3 149 453
- DE-A- 3 523 679

## Beschreibung

Die Erfindung betrifft ein Verfahren zur Herstellung von 1-Oxa-3,8-diaza-4-oxo-spiro[4,5]decan-Verbindungen, die als Lichtschutzmittel für Polymere oder als Zwischenprodukte bei der Herstellung von Kunststoffadditiven verwendet werden können.

Verbindungen der Formel 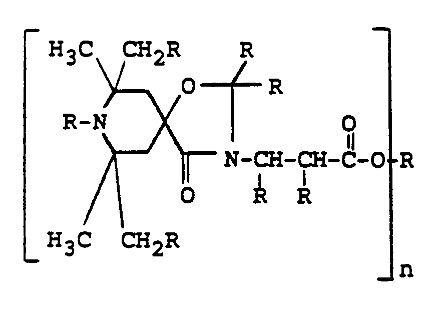
sind bekannt (vgl. DE 31 49 453).

Allerdings ist das in DE 31 49 453 beschriebene Verfahren zu ihrer Herstellung kompliziert, da während der Reaktion das Reaktionsmedium mehrfach gewechselt wird, was zusätzliche Extraktionen und Destillationen bedingt.

In DE 35 24 543 wird ein verbessertes Herstellverfahren für diese Verbindungen beschrieben, das dadurch gekennzeichnet ist, daß die Synthese in einem bei Raumtemperatur flüssigen aromatischen Kohlenwasserstoff erfolgt und zusätzlich zu einem basischen Katalysator noch ein Phasentransferkatalysator eingesetzt wird.

Der in DE 35 24 543 beschriebene Zusatz eines Phasentransferkatalysators bewirkt zwar einen deutlich schnelleren und vollständigeren Umsatz, hat aber als nachteiligen Nebeneffekt eine stärkere Umweltbelastung zur Folge, da der Phasentransferkatalysator bei der Aufarbeitung des Reaktionsgemisches in das Abwasser gelangt. Die Verwendung von Phasentransferkatalysatoren bedeutet im günstigen Fall - bei der Verwendung von Polyethylenglykoldialkylethern - eine Erhöhung der organischen Fracht im Abwasser und damit eine erhöhte Umweltbelastung. Setzt man die in DE 35 24 543 als besonders wirksam beschriebenen quartären Ammonium- oder Phosphoniumhalogenide als Phasentransferkatalysatoren ein, so wird damit nicht nur die organische Fracht im Abwasser erhöht, sondern sogar die Einleitung des Abwassers in eine biologische Kläranlage unmöglich gemacht, da quartäre Ammonium- und Phosphoniumsalze bakterizide Wirkung besitzen und nicht in einer biologischen Kläranlage aufgearbeitet werden können. Das Abwasser muß daher aufwendig als Sondermüll entsorgt werden.

Es bestand somit die Aufgabe, ein Verfahren zu finden, das die eingangs genannten Verbindungen bei möglichst kurzen Reaktionszeiten in möglichst hohen Ausbeuten zur Verfügung stellt, ohne dabei die aus dem Stand der Technik bekannten Nachteile einer zu geringen Umweltverträglichkeit und einer dadurch bedingten aufwendigen Abwasserentsorgung aufzuweisen.

Dies wird erfindungsgemäß dadurch gelöst, daß man zur Herstellung der genannten Verbindungen als Lösemittel einen bei Raumtemperatur flüssigen aromatischen Kohlenwasserstoff und als alleinigen Katalysator eine Verbindung der Formel X verwendet. 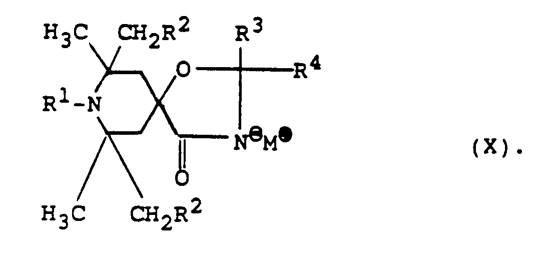
Die vorliegende Erfindung betrifft somit ein Verfahren zur Herstellung von 1-Oxa-3,8-diaza-4-oxo-spiro[4,5]-decan-Verbindungen der Formel I 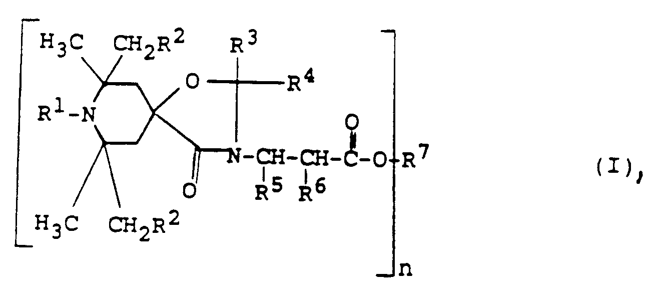
worin
- n: eine ganze Zahl von 1 bis 4 ist,
- R¹: Wasserstoff, C₁-C₄-Alkyl, Benzyl, Allyl, C₂-C₃₀-Alkanoyl, C₃-C₂₀-Alkenoyl, C₇-C₁₁-Aroyl, C₈-C₁₄-Arylalkanoyl oder C₈-C₂₀-Alkylaryl ist,
- R²: Wasserstoff oder C₁-C₄-Alkyl bedeutet,
- R³: Wasserstoff, C₁-C₁₈-Alkyl, C₅-C₁₂-Cycloalkyl, eine Phenyl- oder Naphthylgruppe, die durch Chlor oder C₁-C₄-Alkyl substituiert sein kann, oder eine gegebenenfalls durch C₁-C₄-Alkyl substituierte C₇-C₁₂-Phenylalkylgruppe ist,
- R⁴: Wasserstoff, C₁-C₄-Alkyl, C₅-C₁₂-Cycloalkyl, durch -COOH, Carb-C₁-C₄-alkoxy oder Carbamoyl substituiertes C₁-C₃-Alkenyl, eine Phenyl-, Naphthyl- oder Pyridylgruppe, die durch C₁-C₄-Alkoxy oder C₁-C₄-Alkyl substituiert sein kann, oder eine C₇-C₁₂-Phenylalkylgruppe, die durch C₁-C₄-Alkyl substituiert sein kann, bedeutet, oder
- R³ und R⁴: zusammen mit dem sie bindenden Kohlenstoffatom eine Cycloalkylgruppe, die durch eine bis vier C₁-C₄-Alkylgruppen substituiert sein kann, oder einen Rest der Formel II 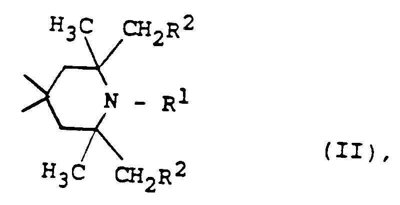 worin R¹ und R² die obengenannte Bedeutung haben, bilden,
- R⁵: Wasserstoff, Methyl, Phenyl oder Carb-C₁-C₂₁-alkoxy ist,
- R⁶: Wasserstoff oder Methyl bedeutet,
- R⁷: bei n = 1
Wasserstoff, C₁-C₂₁-Alkyl, C₂-C₂₂-Alkenyl, C₇-C₁₈-Phenylalkyl, C₅-C₁₂-Cycloalkyl, Phenyl, Naphthyl, C₇-C₁₈-Alkylphenyl, einen Rest der Formel 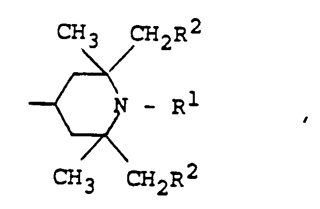 worin R¹ und R² die obige Bedeutung haben, C₂-C₂₀-Alkyl, welches unterbrochen ist durch -O- oder 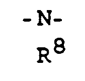 und/oder substituiert durch einen Rest der Formel III 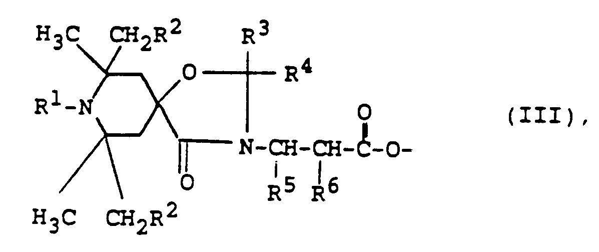 oder durch C₁-C₂₁-Alkylcarboxyl, wobei R¹, R², R³, R⁴, R⁵ und R⁶ die obige Bedeutung haben und
R⁸ Wasserstoff oder C₁-C₁₀-Alkyl ist, bedeutet,
- R⁷: bei n = 2
geradkettiges oder verzweigtes C₁-C₃₀-Alkylen, C₂-C₃₀-Alkenylen, Phenyldialkylen, wobei diese Reste durch -O- oder 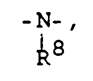 worin R⁸ die obige Bedeutung hat, unterbrochen sein können, bedeutet,
- R⁷: bei n = 3 oder 4
einen Rest der Formeln IV, V, VI oder VII 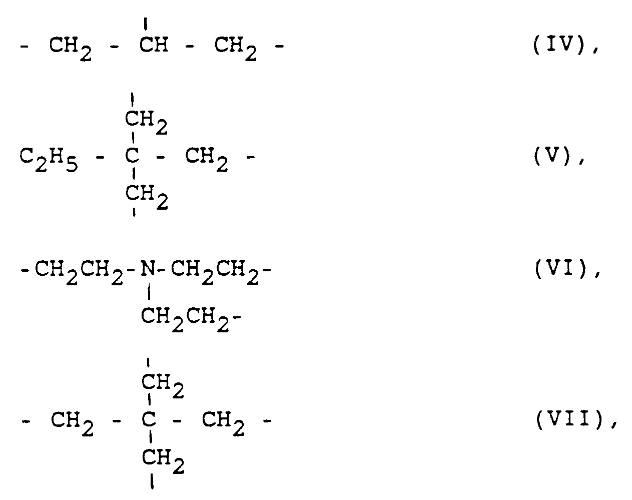 bedeutet,
durch Umsetzung einer Verbindung der Formel VIII 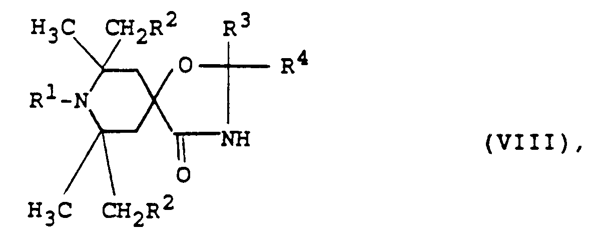
mit einer Verbindung der Formel IX 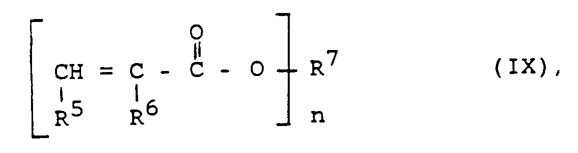
wobei n, R¹, R², R³, R⁴, R⁵, R⁶ und R⁷ die obengenannte Bedeutung haben, in einem inerten Lösemittel bei einer Temperatur von 30 bis 150°C und in Gegenwart eines Katalysators, dadurch gekennzeichnet, daß die Umsetzung in Gegenwart von 1 bis 10 mol-%, bezogen auf die Verbindung VIII, eines Katalysators der Formel (X) 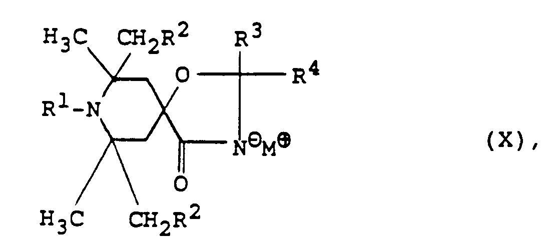
wobei R¹, R², R³ und R⁴ die oben genannte Bedeutung haben und M für ein Alkalimetall steht, in einem bei Raumtemperatur flüssigen aromatischen Kohlenwasserstoff durchgeführt wird.
- R¹: ist bevorzugt Wasserstoff, C₁-C₄-Alkyl, C₂-C₁₈-Alkanoyl, beispielsweise Methyl, Ethyl, Propyl, Butyl, Acetyl, Propionyl, Butyryl, Lauroyl, Stearoyl, besonders bevorzugt Wasserstoff oder einer der genannten Säurereste. Insbesondere ist R¹ Wasserstoff.
- R²: ist bevorzugt Wasserstoff oder C₁-C₄-Alkyl, beispielsweise Methyl, Ethyl, Propyl, Butyl. Insbesondere ist R² Wasserstoff.
- R³ und R⁴: sind unabhängig voneinander C₁-C₁₈-Alkyl, C₅-C₁₂-Cycloalkyl oder Phenyl, beispielsweise Ethyl, Butyl, Octyl, Lauryl, Stearyl, Cyclohexyl, Cyclodecyl, besonders bevorzugt C₁-C₇-Alkyl. Insbesondere sind R³ und R⁴ C₁-C₄-Alkyl, beispielsweise Methyl.
- R³ und R⁴: zusammen mit dem sie bindenden Kohlenstoffatom sind bevorzugt C₅-C₁₂-Cycloalkylen, besonders bevorzugt C₆- bzw. C₁₂-Cycloalkylen, insbesondere Cyclododecylen.
- R⁵: ist bevorzugt Wasserstoff, Methyl oder Phenyl, besonders bevorzugt Wasserstoff.
- R⁶: ist bevorzugt Wasserstoff oder Methyl. Insbesondere ist R⁶ Wasserstoff.
- R⁷: ist bevorzugt C₁-C₂₁-Alkyl, geradkettiges oder verzweigtes C₁-C₃₀-Alkylen, beispielsweise Methyl, Butyl, Octyl, Lauryl, Stearyl, Ethylen, Butylen, Hexylen, besonders bevorzugt C₁-C₁₅-Alkyl. Insbesondere ist R⁷ C₁₂-C₁₄-Alkyl, beispielsweise Lauryl.

Die Ausgangsverbindungen der Formeln VIII und IX sind bekannt und ihre Herstellung ist in der Literatur beschrieben (z.B. DE 31 49 453 und DE 26 06 026).

Geeignete Verbindungen der Formel VIII sind beispielsweise
2-Butyl-7,7,9,9-tetramethyl-1-oxa-3,8-diaza-4-oxo-spiro-[4,5]-decan
2-iso-Butyl-7,7,9,9-tetramethyl-1-oxa-3,8-diaza-4-oxo-spiro-[4,5]-decan
2-Pentyl-7,7,9,9-tetramethyl-1-oxa-3,8-diaza-4-oxo-spiro-[4,5]-decan
2-iso-Pentyl-7,7,9,9-tetramethyl-1-oxa-3,8-diaza-4-oxo-spiro-[4,5]-decan
2-Hexyl-7,7,9,9-tetramethyl-1-oxa-3,8-diaza-4-oxo-spiro-[4,5]-decan
2-Heptyl-7,7,9,9-tetramethyl-1-oxa-3,8-diaza-4-oxo-spiro-[4,5]-decan
2-iso-Heptyl-7,7,9,9-tetramethyl-1-oxa-3,8-diaza-4-oxo-spiro-[4,5]-decan
2-Nonyl-7,7,9,9-tetramethyl-1-oxa-3,8-diaza-4-oxo-spiro-[4,5]-decan
2-iso-Nonyl-7,7,9,9-tetramethyl-1-oxa-3,8-diaza-4-oxo-spiro-[4,5]-decan
2-Undecyl-7,7,9,9-tetramethyl-1-oxa-3,8-diaza-4-oxo-spiro-[4,5]-decan
2-Phenyl-7,7,9,9-tetramethyl-1-oxa-3,8-diaza-4-oxo-spiro-[4,5]-decan
2-(4-Chlor-phenyl)-7,7,9,9-tetramethyl-1-oxa-3,8-diaza-4-oxo-spiro-[4,5]-decan
2-Ethyl-2,7,7,9,9-pentamethyl-1-oxa-3,8-diaza-4-oxo-spiro-[4,5]-decan
2-Propyl-2,7,7,9,9-pentamethyl-1-oxa-3,8-diaza-4-oxo-spiro-[4,5]-decan
2-iso-Propyl-2,7,7,9,9-pentamethyl-1-oxa-3,8-diaza-4-oxo-spiro-[4,5]-decan
2-Butyl-2,7,7,9,9-pentamethyl-1-oxa-3,8-diaza-4-oxo-spiro-[4,5]-decan
2-iso-Butyl-2,7,7,9,9-pentamethyl-1-oxa-3,8-diaza-4-oxo-spiro-[4,5]-decan
2-Pentyl-2,7,7,9,9-pentamethyl-1-oxa-3,8-diaza-4-oxo-spiro-[4,5]-decan
2-Hexyl-2,7,7,9,9-pentamethyl-1-oxa-3,8-diaza-4-oxo-spiro-[4,5]-decan
2-Nonyl-2,7,7,9,9-pentamethyl-1-oxa-3,8-diaza-4-oxo-spiro-[4,5]-decan
2,2,7,7,9,9-Hexamethyl-1-oxa-3,8-diaza-4-oxo-spiro-[4,5]-decan
2,2,7,7,8,9,9-Heptamethyl-1-oxa-3,8-diaza-4-oxo-spiro-[4,5]-decan
2,2-Diethyl-7,7,9,9-tetramethyl-1-oxa-3,8-diaza-4-oxo-spiro-[4,5]-decan
2,2-Dipropyl-7,7,9,9-tetramethyl-1-oxa-3,8-diaza-4-oxo-spiro-[4,5]-decan
2,2-Dibutyl-7,7,9,9-tetramethyl-1-oxa-3,8-diaza-4-oxo-spiro-[4,5]-decan
2-Ethyl-2-pentyl-7,7,9,9-tetramethyl-1-oxa-3,8-diaza-4-oxo-spiro-[4,5]-decan
2,2-Dibenzyl-7,7,9,9-tetramethyl-1-oxa-3,8-diaza-4-oxo-spiro-[4,5]-decan
2,2,4,4-Tetramethyl-7-oxa-3,13-diaza-14-oxo-dispiro-[5,1,4,2]-tetradecan
2,2,4,4-Tetramethyl-7-oxa-3,14-diaza-15-oxo-dispiro-[5,1,5,2]-pentadecan
2,2,4,4-Tetramethyl-7-oxa-3,20-diaza-21-oxo-dispiro-[5,1,11,2]-heneicosan
2,2,7,7,9,9-Hexamethyl-1-oxa-3,8-diaza-4-oxo-8-acetyl-spiro-[4,5]-decan
2,2,4,4-Tetramethyl-7-oxa-3,14-diaza-15-oxo-3-acetyl-dispiro-[5,1,5,2]-pentadecan
2,2,4,4-Tetramethyl-7-oxa-3,20-diaza-21-oxo-3-acetyl-dispiro-[5,1,11,2]-heneicosan
Geeignete Verbindungen der Formel IX sind beispielsweise
Acrylsäuremethylester
Acrylsäureethylester
Acrylsäure-n-butylester
Acrylsäure-iso-butylester
Acrylsäure-tert.-butylester
Acrylsäure-2-ethylhexylester
Acrylsäureoctylester
Acrylsäurelaurylester
Acrylsäuremyristylester
Acrylsäure-2-diethylaminoethylester
Methacrylsäuremethylester
Methacrylsäureethylester
Methacrylsäure-n-butylester
Methacrylsäure-iso-butylester
Methacrylsäure-tert.-butylester
Methacrylsäurelaurylester
Methacrylsäure-cyclohexylester
Methacrylsäureallylester
Methacrylsäure-2-ethoxyethylester
Methacrylsäure-2-dimethylaminoethylester
Crotonsäuremethylester
Crotonsäureethylester
1,4-Butandioldiacrylat
1,6-Hexandioldiacrylat
2-Ethyl-2-hydroxymethyl-1,3-propandiol-triacrylat
Diethylenglykoldiacrylat
Pentaerythrittriacrylat
Pentaerythrittetraacrylat
Ethylenglykoldimethacrylat
1,4-Butandioldimethacrylat
1,6-Hexandioldimethacrylat
Diethylenglykoldimethacrylat
Triethylenglykoldimethacrylat
Tripropylenglykoldiacrylat
Trimethylolpropantrimethacrylat
Acrylsäure-2,2,6,6-tetramethylpiperid-4-yl-ester
Crotonsäure-2,2,6,6-tetramethylpiperid-4-yl-ester
Methacrylsäure-2,2,6,6-tetramethylpiperid-4-yl-ester
Besonders bevorzugt unter den Verbindungen VIII ist 2,2,4,4-Tetramethyl-7-oxa-3,20-diaza-21-oxo-dispiro-[5,1,11,2]-heneicosan, unter den Verbindungen IX Acrylsäurelaurylester.

Für das erfindungsgemäße Verfahren wird als Lösemittel ein bei Raumtemperatur flüssiger aromatischer Kohlenwasserstoff verwendet, wie z.B. Toluol, Xylol oder Mesitylen. Bevorzugt sind die Xylole (o-, m- und p-) und Toluol, insbesondere o-Xylol. Auch Gemische solcher aromatischer Kohlenwasserstoffe können eingesetzt werden.

Der Katalysator der Formel X wird in einer Menge von 1 bis 10 mol-%, bezogen auf eine Verbindung der Formel VIII, vorzugsweise in einer Menge von 1 bis 5 mol-%, insbesondere in einer Menge von 2 bis 4 mol-% eingesetzt. Bei den Katalysatoren der Formel X handelt es sich um die Alkalisalze der Verbindungen VIII, vorzugsweise um deren Lithium-, Natrium- oder Kaliumsalze, insbesondere um Natriumsalze.

Die Herstellung des Katalysators erfolgt in bekannter Weise durch Umsetzung einer Verbindung VIII mit einem Alkalimetall in einem inerten Lösemittel, vorzugsweise in Toluol oder Xylol. Der Katalysator kann nach Abtrennung des Lösemittels als isolierter Feststoff oder ohne Abtrennung des Lösemittels in Form einer Suspension oder Lösung eingesetzt werden.

Besonders vorteilhaft ist es, den Katalysator "in situ hergestellt" zu verwenden. Dazu wird zur Reaktionsmischung, die nur aus dem Lösemittel und einer Verbindung der Formel VIII besteht, ein Alkalimetall in einer Menge von 1 bis 10 Mol-%, bezogen auf die Verbindung VIII, gegeben und die Verbindung VIII zu einem Katalysator gemäß Formel X umgesetzt. Erst wenn das Alkalimetall vollständig reagiert hat, wird als zweite Reaktionskomponente eine Verbindung der Formel IX zugegeben. Die vollständige Umsetzung des zugegebenen Alkalimetalls mit der vorgelegten Verbindung VIII ist hier von entscheidender Bedeutung für eine hohe Ausbeute an den gewünschten Reaktionsprodukten der Formel I. Eine vollständige Umsetzung des Alkalimetalls wird zweckmäßigerweise dadurch sichergestellt, daß das Gemisch aus Lösemittel, Verbindung VIII und Alkalimetall in Abhängigkeit von der Ansatzgröße für eine bestimmte Zeit (vgl. Bsp. 7 und 8) auf Rückflußtemperatur erhitzt wird. Danach wird auf die eigentliche Reaktionstemperatur abgekühlt und die Verbindung der Formel IX zugegeben.

Im Vergleich zum Stand der Technik kann somit beim erfindungsgemäßen Verfahren ohne Ausbeuteverlust und ohne verlängerte Reaktionszeiten auf den für die Aufarbeitung des Reaktionsgemisches nachteilig wirkenden Cokatalysator (Phasentransferkatalysator) verzichtet werden.

Die Verbindung IX wird in einer Menge von 1/n bis 10/n, vorzugsweise 1/n bis 3/n, insbesondere 1/n bis 1,5/n mol, bezogen auf 1 mol der Verbindung VIII, eingesetzt. n hat die oben angegebene Bedeutung.

Die Reaktionstemperatur beträgt 30 bis 150, vorzugsweise 50 bis 120, insbesondere 70 bis 120°C.

Die erfindungsgemäß hergestellten Verbindungen der Formel (I) werden vor allem als Lichtstabilisatoren eingesetzt, beispielsweise für Polyolefine, insbesondere Polyethylen und Polypropylen, Ethylen-Propylen-Copolymere, Polybutylen, sowie Polystyrol, chloriertes Polyethylen, sowie Polyvinylchlorid, Polyester, Polycarbonat, Polymethylmethacrylate, Polyphenylenoxide, Polyamide, Polyurethane, Polypropylenoxid, Polyacetale, Phenol-Formaldehydharze, Epoxidharze, Polyacrylnitril und entsprechende Copolymerisate sowie ABS-Terpolymere. Vorzugsweise verwendet man die erfindungsgemäß hergestellten Verbindungen zum Stabilisieren von Polypropylen, niedermolekularem und hochmolekularem Polyethylen, Ethylen-Propylen-Copolymeren, Polyvinylchlorid, Polyester, Polyamid, Polyurethanen, Polyacrylnitril, ABS, Terpolymeren von Acrylester, Styrol und Acrylnitril, Copolymeren von Styrol und Acrylnitril oder Styrol und Butadien, insbesondere für Polypropylen, Polyethylen, Ethylen-Propylencopolymer oder ABS.

Die erfindungsgemäß hergestellten Verbindungen können auch für die Stabilisierung von Naturstoffen, beispielsweise Kautschuk, verwendet werden, sowie auch für Schmieröle. Weiterhin eignen sie sich auch für die Stabilisierung von Lacken.

Als Lacke kommen alle in der Industrielackierung verwendeten Arten, vorzugsweise Einbrennlacke, infrage, wie sie in DE 3 149 453 aufgezählt sind.

Das Einbringen der erfindungsgemäß hergestellten Verbindungen in die zu schützenden Materialien erfolgt nach an sich bekannten Methoden, wobei auch Monomere oder Vorpolymerisate bzw. Vorkondensate mit diesen Stabilisatoren versehen werden können.

Neben den Verbindungen der Formel (I) können den Kunststoffen noch weitere Stabilisatoren zugesetzt werden. Derartige weitere Verbindungen sind z.B. Antioxydantien auf der Basis sterisch gehinderter Phenole, oder Schwefel oder Phosphor enthaltende Costabilisatoren oder eine Mischung von geeigneten sterisch gehinderten Phenolen und Schwefel und/oder Phosphor enthaltenden Verbindungen. Solche Verbindungen sind z.B. Benzofuranon(2)- und/oder Indolinon(2)verbindungen, sterisch gehinderte Phenole wie β-(4-Hydroxy-3,5-di-t-butylphenyl)-propionsäurestearylester, Tetrakis-[methylen-3(3',5'-di-t-butyl-4-hydroxy-phenyl-) propionat]-methan, 1,3,3-Tris-(2-methyl-4-hydroxy-5-t-butylphenyl)-butan, 1,3,5 Tris-(4-t-butyl-3-hydroxy-2,6-dimethylbenzyl)-1,3,5-triazin-2,4,6-(1H, 3H, 5H)trion, Bis-(4-t-butyl-3-hydroxy-2,6-dimethylbenzyl)dithiolterephthalat, Tris-(3,5-di-t-butyl-4-hydroxybenzyl) isocyanurat, Triester der β-(4-Hydroxy-3,5-di-t-butylphenyl)propionsäure mit 1,3,4-Tris-(2-hydroxyethyl)-1,3,5-triazin 2,4,6-(1H, 3H, 5H)trion, Bis-[3,3-bis-(4'-hydroxy-3-t-butylphenyl)-butansäure]-glycolester, 1,3,5-Trimethyl-2,4,6-tris-(3,5-di-t-butyl-4-hydroxybenzyl-)benzol, 2,2'-Methylen-bis-(4-methyl-6-t-butylphenyl)-terephthalat, 4,4-Methylen-bis-(2,6-di-t-butylphenol), 4,4'-Butyliden-bis-(-t-butyl-meta-kresol), 4,4-Thio-bis-(2-t-butyl-5-methyl-phenol), 2,2'-Methylen-bis-(4-methyl-6-t-butyl-phenol). Auch antioxidativ wirkende Co-Stabilisatoren können zugegeben werden, wie z.B. schwefelhaltige Verbindungen, z.B. Distearylthiodipropionat, Dilaurylthiodipropionat, Tetrakis-(methylen-3-hexyl-thiopropionat)-methan, Tetrakis-(methylen-3-dodecylthiopropionat)-methan und Dioctadecyldisulfid oder phosphorhaltige Verbindungen wie z.B. Trisnonylphenylphosphit; 4,9-Distearyl-3,5,8,10-tetraoxadiphosphaspiroundecan, Tris-(2,4-t-butylphenyl)-phosphit oder Tetrakis-(2,4 di-t-butylphenyl)-4,4'-biphenylen-diphosphonit.

Die Verbindungen der Formel I und deren oben erwähnte Mischungen kann man auch in Gegenwart weiterer Additive verwenden. Solche sind an sich bekannt und gehören z.B. zur Gruppe der Aminoacrylverbindungen, der UV-Absorber und Lichtschutzmittel wie die 2-(2'-Hydroxyphenyl)-benztriazole, 2-Hydroxybenzophenone, 1,3-Bis(2'-hydroxybenzoyl)-benzole, Salicylate, Zimtsäureester, Ester von gegebenenfalls substituierten Benzoesäuren, sterisch gehinderte Amine, Oxalsäurediamide.

Die Anwendungsmenge der erfindungsgemäß hergestellten Verbindungen der Formel I beträgt 0,01-5 Gew.-% bei Kunststoffen, 20 bis 80 Gew.-% bei Stabilisatorkonzentraten und 0,02 bis 5 Gew.-% bei Lacken.

Die nachfolgenden Beispiele dienen zur Erläuterung der vorliegenden Erfindung:

### Vergleichsbeispiel A (gemäß dem Verfahren nach DE 3 524 543)

91,1 g (0,25 mol) 2,2,4,4-Tetramethyl-7-oxa-3,20-diazadispiro-[5.1.11.2]-heneicosan-21-on in 100 ml Toluol wurden auf 80°C erhitzt. Dann wurden 0,30 g (0,013 mol) Natrium, 1,5 g Triethylbenzylammoniumchlorid und 76,5 g (0,30 mol) Laurylacrylat (technisches Gemisch von ca. 55 bis 58 % C12-Ester und ca. 37 bis 40 % C14-Ester) zugegeben und das Gemisch 4 h bei 80°C gerührt. Anschließend wurde der Ansatz dreimal mit je 100 ml Wasser ausgerührt und von der organischen Phase das Lösemittel abdestilliert. Man erhielt 168 g Produkt (leicht gelbe hochviskose Flüssigkeit) mit einem Restgehalt von 0,7 Gew.-% (nach GC) 2,2,4,4-Tetramethyl-7-oxa-3,20-diazadispiro-[5.1.11.2]-heneicosan-21-on.

### Vergleichsbeispiel B

Analog Vergleichsbeispiel A, jedoch mit 1,5 g Tetrabutylphosphoniumchlorid (anstelle von Triethylbenzylammoniumchlorid).

Man erhielt 167 g Produkt (leicht gelbe hochviskose Flüssigkeit) mit einem Restgehalt von 1,1 Gew.-% 2,2,4,4-Tetramethyl-7-oxa-3,20-diazadispiro-[5.1.11.2]-heneicosan-21-on.

### Beispiel 1

91,1 g (0,25 mol) 2,2,4,4-Tetramethyl-7-oxa-3,20-diazodispiro-[5.1.11.2]-heneicosan-21-on in 100 ml Toluol wurden auf 80°C erhitzt. Dann wurden 3,0 g (0,008 mol) Natriumsalz von 2,2,4,4-Tetramethyl-7-oxa-3,20-diazadispiro-[5.1.11.2]-heneicosan-21-on ("Natriumamid") und 76,5 g (0,30 mol) Laurylacrylat zugegeben und der Ansatz 4 h bei 80°C gerührt. Anschließend wurde das Gemisch dreimal mit je 100 ml Wasser ausgerührt und von der organischen Phase das Lösemittel abdestilliert.

Man erhielt 171 g Produkt (farblose, hochviskose Flüssigkeit) mit einem Restgehalt von 0,9 Gew.-% 2,2,4,4-Tetramethyl-7-oxa-3,20-diazadispiro-[5.1.11.2]-heneicosan-21-on.

### Beispiel 2

Analog Beispiel 1, jedoch wurden 5,0 g (0,013 mol) Natriumsalz von 2,2,4,4-Tetramethyl-7-oxa-3,20-diazadispiro-[5.1.11.2]-heneicosan-21-on als Katalysator eingesetzt und der Ansatz nur 2 h bei 80°C gerührt.

Man erhielt 171 g Produkt (farblose, hochviskose Flüssigkeit) mit einem Restgehalt von 0,9 Gew.-% 2,2,4,4-Tetramethyl-7-oxa-3,20-diazadispiro-[5.1.11.2]-heneicosan-21-on.

### Beispiel 3

Analog Beispiel 1, jedoch wurden 1,0 g (0,003 mol) Natriumsalz von 2,2,4,4-Tetramethyl-7-oxa-3,20-diazadispiro-[5.1.11.2]heneicosan-21-on als Katalysator eingesetzt und der Ansatz 1,5 h bei 120°C gerührt.

Man erhielt 166 g Produkt (farblose, hochviskose Flüssigkeit) mit einem Restgehalt von 1,3 Gew.-% 2,2,4,4-Tetramethyl-7-oxa-3,20-diazadispiro-[5.1.11.2]-heneicosan-21-on.

### Beispiele 4 bis 6

Analog Beispiel 1, jedoch wurde anstelle von Toluol o-Xylol als Lösemittel verwendet.

| Katalysator | Endprodukt (g) | Restgehalt an 2,2,4,4-Tetramethyl-7-oxa-3,20-diazadispiro-[5.1.11.2]-heneicosan-21-on in Gew.-% |
|---|---|---|
| 3,0 g Natriumsalz | 169 | 0,6 |
| 3,0 g Kaliumsalz | 170 | 0,6 |
| 2,0 g Lithiumsalz | 170 | 0,7 |

### Beispiel 7 (dieses Beispiel zeigt den Einsatz von "in situ hergestelltem" Katalysator)

Zu 91,1 g (0,25 mol) 2,2,4,4-Tetramethyl-7-oxa-3,20-diazadispiro-[5.1.11.2]-heneicosan-21-on in 100 ml o-Xylol wurden 0,18 g (0,008 mol) Natrium gegeben und die Mischung solange auf Rückfluß erhitzt, bis das Natrium vollständig wegreagiert war (ca. 1 h). Dann wurde die Reaktionsmischung auf 80°C abgekühlt, 76,5 g (0,30 mol) Laurylacrylat zugegeben und der Ansatz 4 h bei 80°C gerührt. Anschließend wurde das Gemisch dreimal mit je 100 ml Wasser ausgerührt und von der organischen Phase das Lösemittel abdestilliert.

Man erhielt 168 g Produkt mit einem Restgehalt von 0,4 Gew.-% 2,2,4,4-Tetramethyl-7-oxa-3,20-diazadispiro-[5.1.11.2]-heneicosan-21-on.

### Beispiel 8 (Synthese des Katalysators (Natriumsalz))

145,6 g (0,40 mol) 2,2,4,4-Tetramethyl-7-oxa-3,20-diazadispiro-[5.1.11.2]-heneicosan-21-on und 9,2 g (0,40 mol) Natrium wurden in 500 ml Toluol 24 h bei Rückflußtemperatur gerührt. Dann wurde der Ansatz heiß filtriert und vom Filtrat das Lösemittel abgezogen. Man erhielt 150,9 g (98 % d. Theorie) Katalysator als weißen Feststoff mit einem Schmp. von 235°C.

## Patentansprüche

1. Verfahren zur Herstellung von 1-Oxa-3,8-diaza-4-oxo-spiro[4,5]-decan-Verbindungen der Formel I 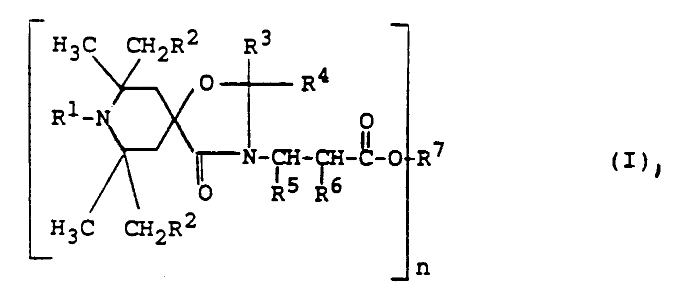 worin
n eine ganze Zahl von 1 bis 4 ist,
R¹ Wasserstoff, C₁-C₄-Alkyl, Benzyl, Allyl, C₂-C₃₀-Alkanoyl, C₃-C₂₀-Alkenoyl, C₇-C₁₁-Aroyl, C₈-C₁₄-Arylalkanoyl oder C₈-C₂₀-Alkylaryl ist,
R² Wasserstoff oder C₁-C₄-Alkyl bedeutet,
R³ Wasserstoff, C₁-C₁₈-Alkyl, C₅-C₁₂-Cycloalkyl, eine Phenyl- oder Naphthylgruppe, die durch Chlor oder C₁-C₄-Alkyl substituiert sein kann, oder eine gegebenenfalls durch C₁-C₄-Alkyl substituierte C₇-C₁₂-Phenylalkylgruppe ist,
R⁴ Wasserstoff, C₁-C₄-Alkyl, C₅-C₁₂-Cycloalkyl, durch -COOH, Carb-C₁-C₄-alkoxy oder Carbamoyl substituiertes C₁-C₃-Alkenyl, eine Phenyl-, Naphthyl- oder Pyridylgruppe, die durch C₁-C₄-Alkoxy oder C₁-C₄-Alkyl substituiert sein kann, oder eine C₇-C₁₂-Phenylalkylgruppe, die durch C₁-C₄-Alkyl substituiert sein kann, bedeutet, oder
R³ und R⁴ zusammen mit dem sie bindenden Kohlenstoffatom eine Cycloalkylgruppe, die durch eine bis vier C₁-C₄-Alkylgruppen substituiert sein kann, oder einen Rest der Formel II 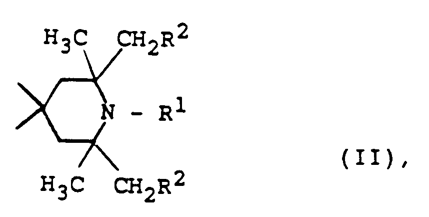 worin R¹ und R² die obengenannte Bedeutung haben, bilden
R⁵ Wasserstoff, Methyl, Phenyl oder Carb-C₁-C₂₁-alkoxy ist,
R⁶ Wasserstoff oder Methyl bedeutet,
R⁷ bei n = 1
Wasserstoff, C₁-C₂₁-Alkyl, C₂-C₂₂-Alkenyl, C₇-C₁₈-Phenylalkyl, C₅-C₁₂-Cycloalkyl, Phenyl, Naphthyl, C₇-C₁₈-Alkylphenyl, einen Rest der Formel 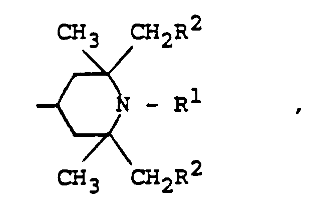 worin R¹ und R² die obige Bedeutung haben, C₂-C₂₀-Alkyl, welches unterbrochen ist durch -O- oder 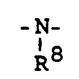 und/oder substituiert durch einen Rest der Formel III 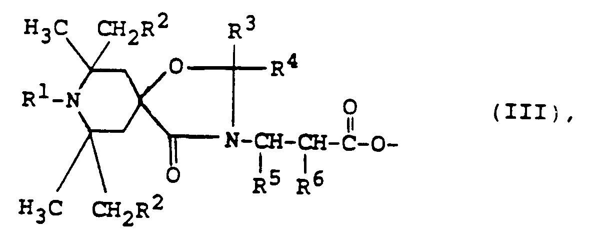 oder durch C₁-C₂₁-Alkylcarboxyl, wobei R¹, R², R³, R⁴, R⁵ und R⁶ die obige Bedeutung haben und
R⁸ Wasserstoff oder C₁-C₁₀-Alkyl ist, bedeutet,
R⁷ bei n = 2
geradkettiges oder verzweigtes C₁-C₃₀-Alkylen, C₂-C₃₀-Alkenylen, Phenyldialkylen, wobei diese Reste durch -O- oder 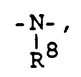 worin R⁸ die obige Bedeutung hat, unterbrochen sein können, bedeutet,
R⁷ bei n = 3 oder 4
einen Rest der Formeln IV, V, VI oder VII 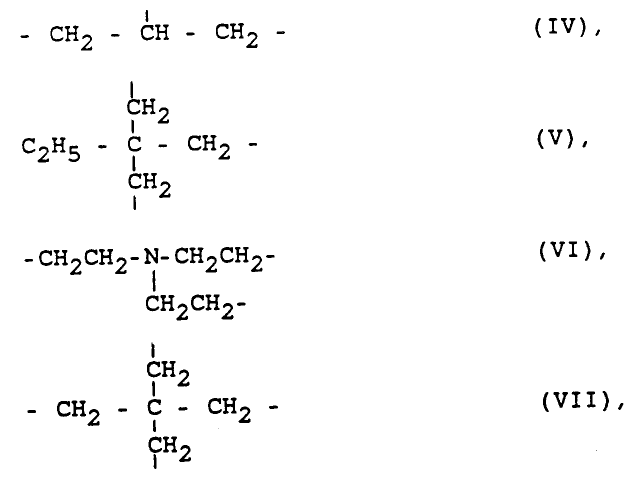 bedeutet,
durch Umsetzung einer Verbindung der Formel VIII 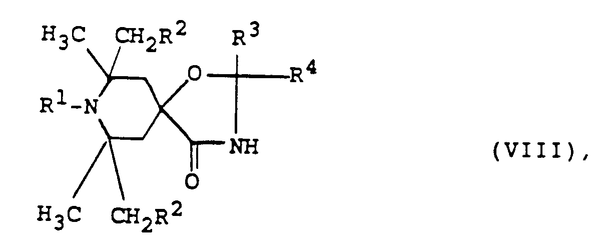 mit einer Verbindung der Formel IX 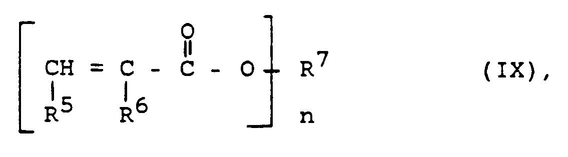 wobei n, R¹, R², R³, R⁴, R⁵, R⁶ und R⁷ die obengenannte Bedeutung haben, in einem inerten Lösemittel bei einer Temperatur von 30 bis 150°C und in Gegenwart eines Katalysators, dadurch gekennzeichnet, daß die Umsetzung in Gegenwart von 1 bis 10 mol-%, bezogen auf die Verbindung VIII, eines Katalysators der Formel (X) 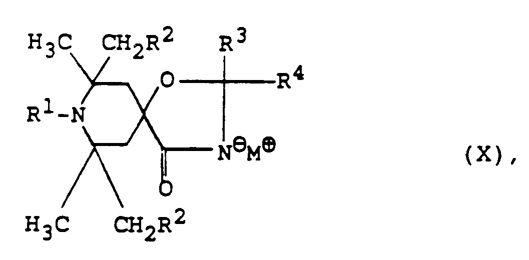 wobei R¹, R², R³ und R⁴ die oben genannte Bedeutung haben und M für ein Alkalimetall steht, in einem bei Raumtemperatur flüssigen aromatischen Kohlenwasserstoff durchgeführt wird.

2. Verfahren gemäß Anspruch 1, dadurch gekennzeichnet, daß der aromatische Kohlenwasserstoff Toluol oder Xylol ist.

3. Verfahren gemäß Anspruch 1, dadurch gekennzeichnet, daß als Katalysator ein Lithium-, Natrium- oder Kaliumsalz einer Verbindung der Formel VIII verwendet wird.

4. Verfahren gemäß Anspruch 1, dadurch gekennzeichnet, daß als Katalysator das Natriumsalz einer Verbindung der Formel VIII verwendet wird.

5. Verfahren gemäß Anspruch 1, dadurch gekennzeichnet, daß der Katalysator im Reaktionsgemisch nach vollständiger Reaktion des Alkalimetalls in situ vor Zugabe der Verbindung der Formel IX hergestellt wird.

6. Verfahren gemäß Anspruch 1, dadurch gekennzeichnet, daß die Verbindung der Formel VIII 2,2,4,4-Tetramethyl-7-oxo-3,20-diaza-21-oxo-dispiro-[5,1,11,2]-heneicosan ist.

7. Verfahren gemäß Anspruch 1, dadurch gekennzeichnet, daß die Verbindung der Formel IX Acrylsäurelaurylester ist.

## Claims

1. Process for the manufacture of 1-oxa-3,8-diaza-4-oxo-spiro[4,5] decane compounds of formula I 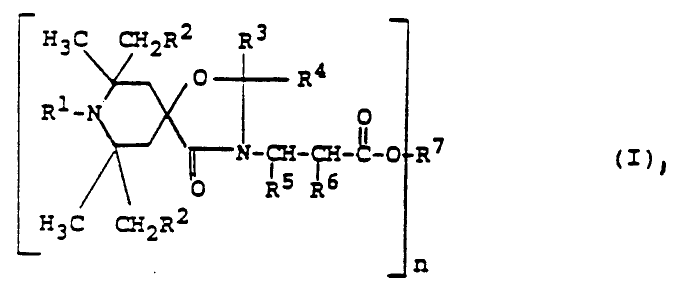 in which
n is an integer from 1 to 4,
R¹ is hydrogen, C₁-C₄-alkyl, benzyl, allyl, C₂-C₃₀-alkanoyl, C₃-C₂₀-alkenoyl, C₇-C₁₁-aroyl, C₈-C₁₄-arylalkanoyl or C₈-C₂₀-alkylaryl,
R² is hydrogen or C₁-C₄-alkyl,
R³ is hydrogen, C₁-C₁₈-alkyl, C₅-C₁₂-cycloalkyl, a phenyl or naphthyl group that can be substituted for chlorine or C₁-C₄-alkyl, or a C₇-C₁₂-phenylalkyl group that may be substituted for C₁-C₄-alkyl,
R⁴ is hydrogen, C₁-C₄-alkyl, C₅-C₁₂-cycloalkyl, C₁-C₃-alkenyl substituted for -COOH, carb-C₁-C₄-alkoxy or carbamoyl, a phenyl, naphthyl or pyridyl group that can be substituted for C₁-C₄-alkoxy or C₁-C₄-alkyl, or a C₇-C₁₂-phenylalkyl group that can be substituted for C₁-C₄-alkyl, or
R³ and R⁴ together with the carbon atom linking them form a cycloalkyl group that can be substituted for one to four C₁-C₄-alkyl groups, or a radical of formula II 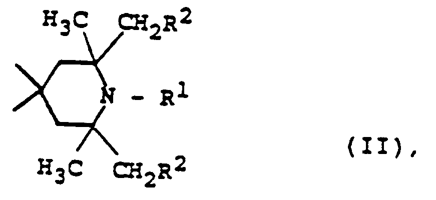 in which R¹ and R² have the above-mentioned meanings,
R⁵ is hydrogen, methyl, phenyl or carb-C₁-C₂₁-alkoxy,
R⁶ is hydrogen or methyl,
R⁷ where n = 1
is hydrogen, C₁-C₂₁-alkyl, C₂-C₂₂-alkenyl, C₇-C₁₈-phenylalkyl, C₅-C₁₂-cycloalkyl, phenyl, naphthyl, C₇-C₁₈-alkylphenyl, a radical of the formula 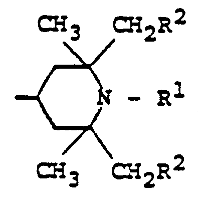 in which R¹ and R² have the above-mentioned meanings, C₂-C₂₀-alkyl that is interrupted by -O- or 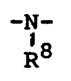 and/or substituted for a radical of formula III 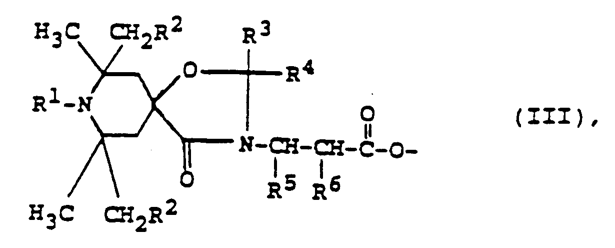 or by C₁-C₂₁-alkylcarboxyl, in which R¹, R², R³, R⁴, R⁵ and R⁶ have the above-mentioned meanings and R⁸ is hydrogen or C₁-C₁₀-alkyl,
R⁷ where n = 2
is straight-chain or branched C₁-C₃₀-alkylene, C₂-C₃₀-alkenylene, phenyldialkylene, where these radicals can be interrupted by -O- or 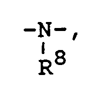 in which R⁸ has the above-mentioned meaning,
R⁷ where n = 3 or 4
is a radical of formula IV, V, VI or VII 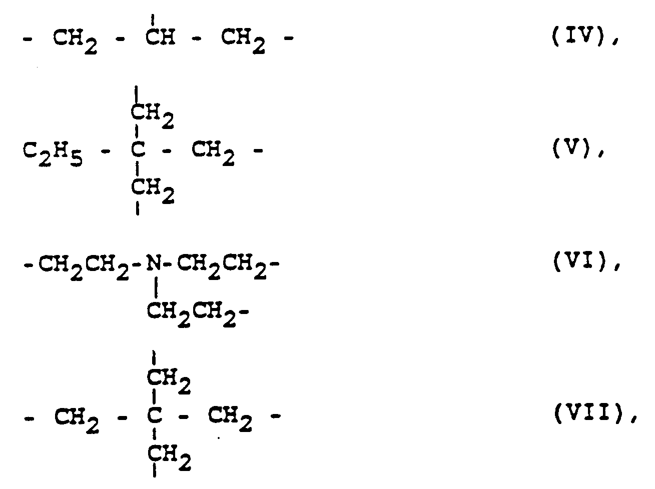
by reacting a compound of formula VIII 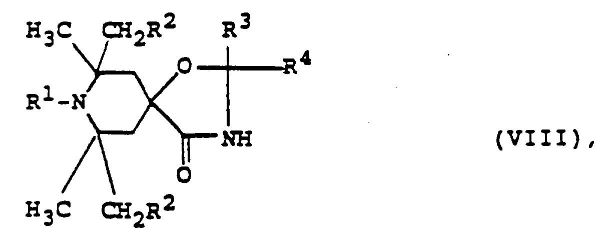 with a compound of formula IX 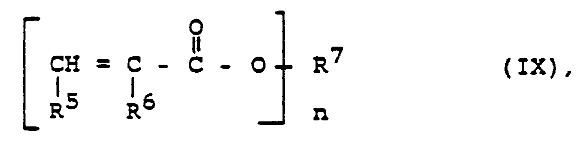 in which n, R¹, R², R³, R⁴, R⁵, R⁶ and R⁷ have the above-mentioned meanings, in an inert solvent at a temperature of 30 to 150°C and in the presence of a catalyst, characterized by the fact that the reaction is carried out in the presence of 1 to 10 % by mol, relative to compound VIII, of a catalyst of formula (X) 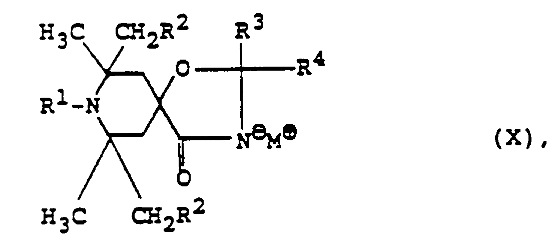 in which R¹, R², R³ and R⁴ have the above-mentioned meanings and M is an alkali metal, in an aromatic hydrocarbon that is liquid at room temperature.

2. The process in accordance with claim 1, characterized by the fact that the aromatic hydrocarbon is toluene or xylene.

3. The process in accordance with claim 1, characterized by the fact that the catalyst used is a lithium, sodium or potassium salt of a compound of formula VIII.

4. The process in accordance with claim 1, characterized by the fact that the catalyst used is the sodium salt of a compound of formula VIII.

5. The process in accordance with claim 1, characterized by the fact that the catalyst is produced in situ in the reaction mixture after complete reaction of the alkali metal before addition of the compound of formula IX.

6. The process in accordance with claim 1, characterized by the fact that the compound of formula VIII is 2,2,4,4-tetramethyl-7-oxa-3,20-diaza-21-oxo-dispiro-[5,1,11,2]-heneicosane.

7. The process in accordance with claim 1, characterized by the fact that the compound of formula IX is acrylic acid lauryl ester.

## Revendications

1. Procédé de préparation de composés 1-oxa-3,8-diaza-4-oxo-spiro[4,5]-décane de formule I 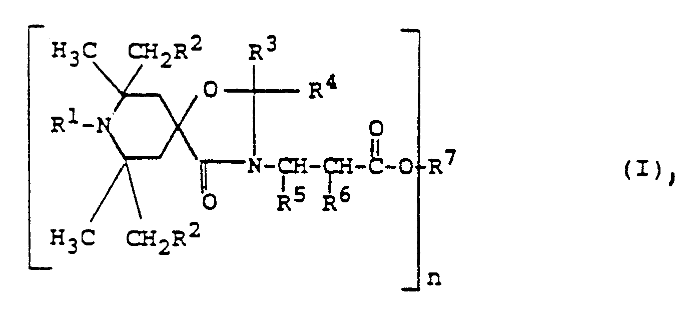 dans laquelle
n est un nombre entier de 1 à 4,
R¹ est un hydrogène ou un alkyle en C₁-C₄, un benzyle, un allyle, un alcanoyle en C₂-C₃₀, un alcénoyle en C₃-C₂₀, un aroyle en C₇-C₁₁, un aryl-alcanoyle en C₈-C₁₄ ou alkylaryle en C₈-C₂₀,
R² signifie un hydrogène ou un alkyle-C₁-C₄,
R³ est un hydrogène ou un alkyle-C₁-C₁₈, cycloalkyle-C₅-C₁₂, un groupe phényle ou naphtyle pouvant avoir comme substituants du chlore ou un alkyle-C₁-C₄, ou bien un groupe phénylalkyle-C₇-C₁₂ éventuellement substitué par un alkyle-C₁-C₄,
R⁴ signifie un hydrogène, un C₁-C₄-alkyle, un C₅-C₁₂-cycloalkyle, un C₁-C₃-alcényle substitué par un -COOH, un Carb-alcoxy-C₁-C₄ ou un carbamoyle, un groupe phényle, un groupe naphtyle ou un groupe pyridyle pouvant avoir comme substituants un alcoxy-C₁-C₄, ou un alkyle-C₁-C₄, ou un groupe phénylalkyle-C₇-C₁₂ pouvant être substitué par un alkyle-C₁-C₄, ou bien
R³ et R⁴ forment ensemble et avec l'atome de carbone qui les relie un groupe cycloalkyle pouvant avoir comme substituants de un à quatre groupes alkyles en C₁-C₄, ou un radical de formule II 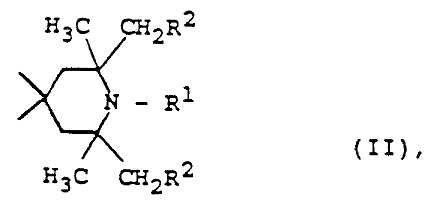
dans laquelle
R¹ et R² ont les significations précédentes,
R⁵ est un hydrogène, un méthyle, un phényle ou un carbalcoxy en C₁-C₂₁,
R⁶ signifie un hydrogène ou un méthyle,
R⁷ si n = 1,
signifie un hydrogène, un alkyle-C₁-C₂₁, un alcényle-C₂-C₂₂, un phénylalkyle-C₇-C₁₈, un cycloalkyle-C₅-C₁₂, un phényle, un naphtyle, un alkylphényle-C₇-C₁₈, un radical de formule 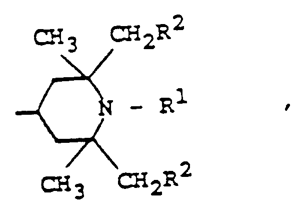 dans laquelle R¹ et R² ont les significations précédentes, ou un alkyle-C₂-C₂₀ interrompu par -O- ou 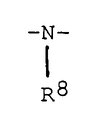 et/ou substitué par un radical de formule III 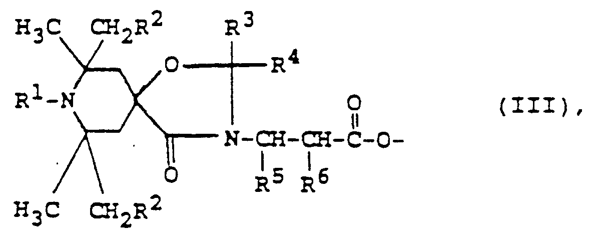 ou par un alkylcarboxyle-C₁-C₂₁, R¹, R², R³, R⁴, R⁵ et R⁶ ayant les significations ci-dessus indiquées et R⁸ étant un hydrogène ou un alkyle-C₁-C₁₀,
si n = 2,
R⁷ désigne un alkylène en C₁-C₃₀, un alcénylène en C₂-C₃₀ ou un phényldialkylène linéaires ou ramifiés et pouvant être interrompus par -O- ou 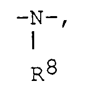 dans lequel R⁸
a la signification ci-dessus,
si n = 3 ou 4,
R⁷ désigne un radical de formule IV, V, VI ou VII 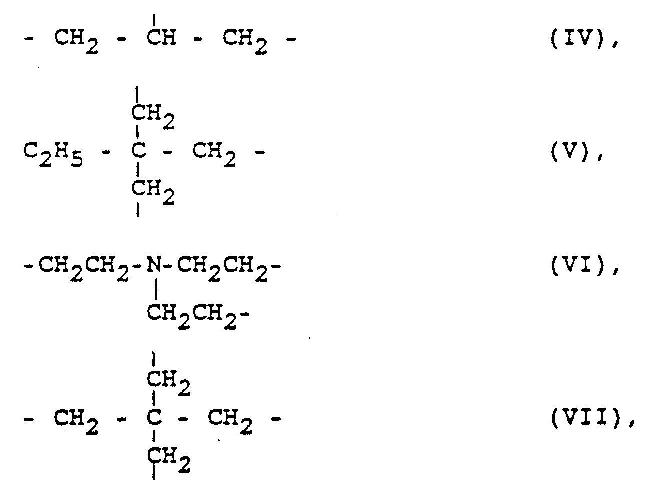 par réaction d'un composé de formule VIII 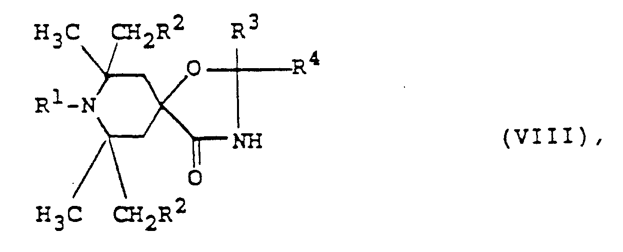 avec un composé de formule IX 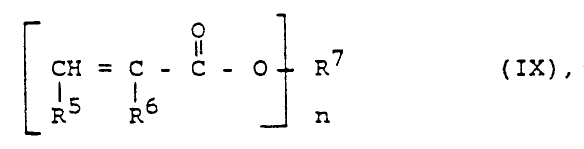 n, R¹, R², R³, R⁴, R⁵, R⁶ et R⁷ ayant les significations précédemment indiquées, dans un solvant inerte à une température de 30 à 150°C et en présence d'un catalyseur, procédé caractérisé en ce que l'on effectue la réaction en présence de 1 à 10 % en mole, par rapport au composé VIII, d'un catalyseur de formule X 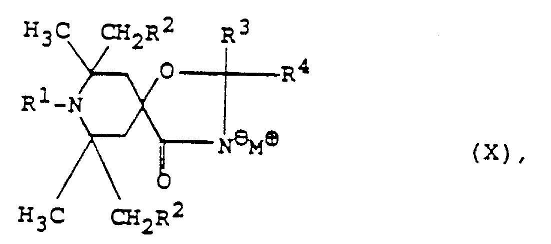 R¹, R², R³ et R⁴ ayant les significations précédentes et M désignant un métal alcalin, dans un hydrocarbure aromatique liquide à la température ambiante.

2. Procédé selon la revendication 1, caractérisé en ce que l'hydrocarbure aromatique est le toluène ou un xylène.

3. Procédé selon la revendication 1, caractérisé en ce que l'on utilise comme catalyseur un sel de lithium, sodium ou potassium d'un composé de formule VIII.

4. Procédé selon la revendication 1, caractérisé en ce que l'on utilise comme catalyseur le sel sodique d'un composé de formule VIII.

5. Procédé selon la revendication 1, caractérisé en ce que le catalyseur est formé in-situ dans le mélange de réaction après réaction totale du métal alcalin, avant l'addition du composé de formule IX.

6. Procédé selon la revendication 1, caractérisé en que le composé de formule VIII est le 2,2,4,4-tétraméthyl-7-oxa-3,20-diaza-21-oxo-dispiro-[5,1,11,2]-heneicosane.

7. Procédé selon la revendication 1, caractérisé en ce que le composé de formule IX est l'acrylate de lauryle.
